# EUROPEAN PATENT SPECIFICATION

(11) **EP 0 524 732 B1**
(45) Date of publication and mention of the grant of the patent: **08.04.1998**
(21) Application number: 92305897.8
(22) Date of filing: 26.06.1992
(51) Int. Cl.: A61K 35/74, C12N 1/20

(54) **Composition containing lactic acid bacteria for preventing dental caries**
Milchsäurebakterien enthaltende Zubereitung zum Schutz von Zahnfäulen
Composition contenant des bactéries lactiques pour la prévention de caries dentaires

(30) Priority: 28.06.1991 JP 158033/91
(43) Date of publication of application: 27.01.1993
(73) Proprietor: Kabushiki Kaisha Ohmori Kikaku, Tokyo 107 (JP)
(72) Inventor: Matsushiro, Aizo, Suita, Osaka 565 (JP)
(74) Representative: Rackham, Anthony Charles

(56) References cited:
- EP-A- 0 195 672
- CHEMICAL ABSTRACTS, vol. 114, no. 11, 18 March 1991, Columbus, Ohio, US; abstract no. 97962y, P. TOWNSEND-LAWMAN ET AL. 'MULTILEVEL CONTROL OF EXTRACELLULAR SUCROSE METABOLISM IN STREPTOCOCCUS SALIVARIUS BY SUCROSE.' page 364 ;

## Description

The present invention relates to a composition containing a lactic acid bacteria, especially a composition having the properties of preventing dental caries and also regulating an intestinal condition, and to a method for producing such a composition.

A lactic acid beverage, a food such as a yogurt containing lactic acid bacteria, and a medicine such as BIOFERMIN (Trade Mark) containing lactic acid bacteria are already in use and widely accepted. Most of these composition contain lactic acid bacteria such as Streptococcus lactis, Streptococcus faecalis, Lactobacillus casei, Lactobacillus acidophilus or Lactobacillus bifidus. These compositions are characterized by their ability to regulate intestinal conditions through the property of the lactic acid bacteria which tend to remain as a human intestinal flora for a while once ingested.

Since the human digestive organs extend from the mouth to the anus through the stomach and the intestine, to acquire an effect such as dental caries prevention in addition to the regulation of the intestinal tract which can be derived only from enteric bacteria, the bacteria must be capable of multiplying and living in the oral cavity.

With regard to dental caries prevention, Japanese Patent Provisional Publication Nos. 62-25, 63-185381 and 63-301788, disclose the use of Streptococcus sanguis prepared by transformation using a genetic engineering technique for producing dextranase and the glucanase which are able to degrade the insoluble glucan which causes the formation of dental plaque.

According to the present invention there is provided a composition comprising a live and native Streptococcus salivarius which is capable of staying in the human oral cavity, of producing dextranase and of producing in a medium a crater-form colony.

The invention also relates to the said live and native Streptococcus salivarius having the properties noted above. The preferred Streptococcus salivarius is Streptococcus salivarius M-33 (FERM BP-3885).

The invention relates to a composition, particularly for oral ingestion, which prevents dental caries together with the conventional property of "regulating the intestinal condition". The composition can be provided as a food, a medicine or the like. The bacteria noted above can be combined with other conventional lactic acid bacteria.

There are various bacteria which are indigenous to the oral cavity amongst which the following bacterial species are classified as lactic acid bacteria (Bergey's Manual of Systematic Bacteriology vol.2 ; Williams & Wilkins, 1986, Baltimore, London, Los Angeles, Sydney):
Streptococcus salivarius,
Streptococcus sanguis,
Streptococcus mitior,
Streptococcus milleri,
Streptococcus mutans,
Streptococcus rattus,
Streptococcus cricetus,
Streptococcus sobrinus,
Streptococcus ferus,
Streptococcus oralis,
Streptococcus mitis.

Among the bacteria listed above, Streptococcus salivarius is harmless to the human species and does not produce insoluble glucan which causes formation of dental plaque.

Streptococcus salivarius is a bacteria which produces a soluble fructan (levan) and, according to the species of the strain, produces an insoluble glucan (dextrans).

Therefore, strains of Streptococcus salivarius, which produce the dextranase and which are capable of degrading insoluble glucan and do not produce insoluble glucan at all, are useful for preventing dental caries.

In order to get such strains from those available from the principal Strain Preservation Institution of Japan, ten strains of Streptococcus salivarius were selected and examined with respect to their productivity of dextranase and insoluble glucan.

### Example 1: Examination of Dextranase Activity of Streptococcus salivarius

The ten strains of Streptococcus salivarius were each inoculated on to Mitis-salivarius Agar (Difco) to which had been added 1% Chapman Solution thereto, and the shape of colony formed was examined. The results are listed below in Table 1.

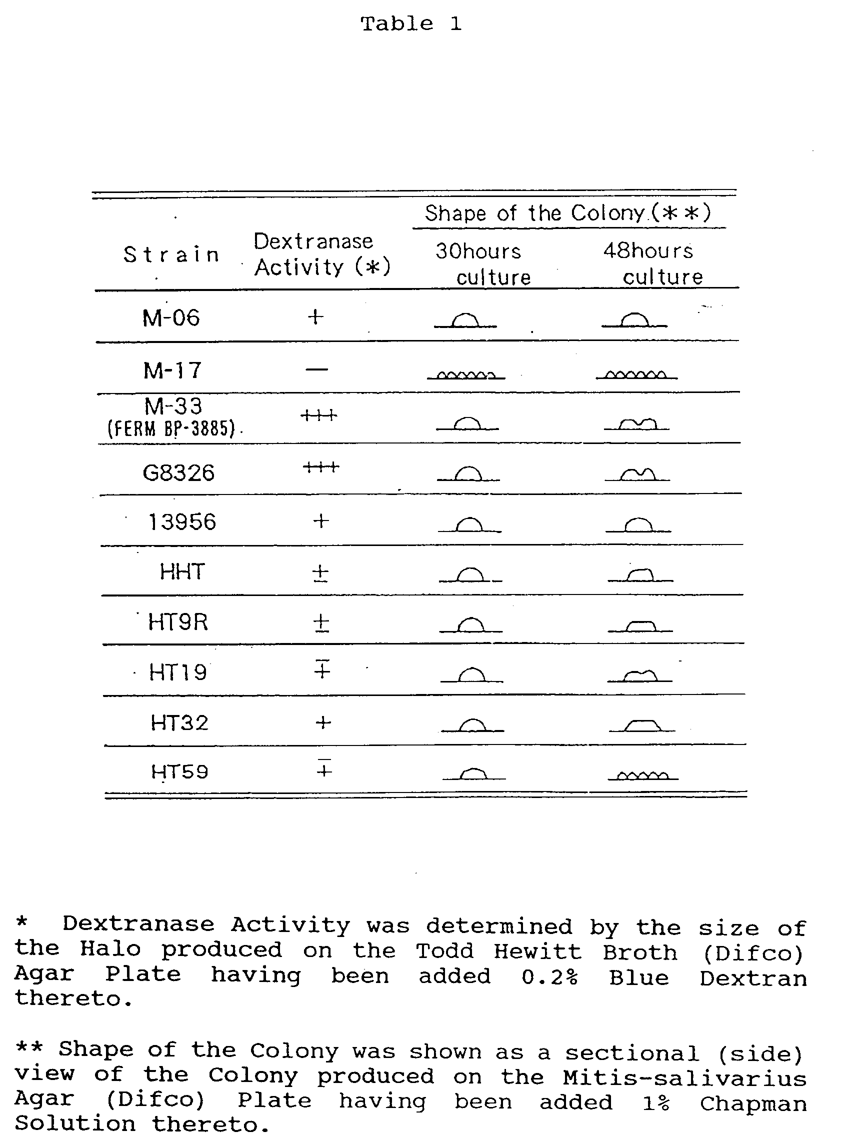

The ten strains were then inoculated on to Todd Hewitt Broth (Difco) to which had been added 0.2% Blue Dextran and the approximate potency of the dextranase productivity was determined based on the size of the transparent halo formed two days after inoculation. Although the conventional knowledge on the dextranase productivity of Streptococcus salivarius was unclear, the results shown in Table 1 indicate that the dextranase productivity of Streptococcus salivarius is different for different strains.

As disclosed in Table 1, Streptococcus salivarius M-33 (FERM BP-3885) and Streptococcus salivarius G8326 produce dextranase remarkably whilst Streptococcus salivarius M-17 did not produce any dextranase. Other strains produce some dextranase, but their enzymatic activity was weak.

Streptococcus salivarius M-33 has remarkable dextranase productivity and the potency thereof, judged from the diameter of the halo, is the same level of the Streptococcus sanguis (pMNK-4) constructed by genetic engineering techniques and used as a control. Since the level of dextranase production of Streptococcus salivarius is substantially identical with that of the control, Streptococcus salivarius M-33 is useful for degrading dental plaque and was, therefore, employed in the following experiments.

Streptococcus salivarius M-33 has been deposited in Fermentation Research Institute, of 1-3, Higashi 1 chome, Tsukuba-shi Ibaraki-ken 305, Japan., an International Depositary Authority under the Budapest Treaty, on the 5th June 1992 under Accession Number FERM BP-3885.

### Example 2: Examination on the Degradation Ability of Dental Plaque by Streptococcus salivarius

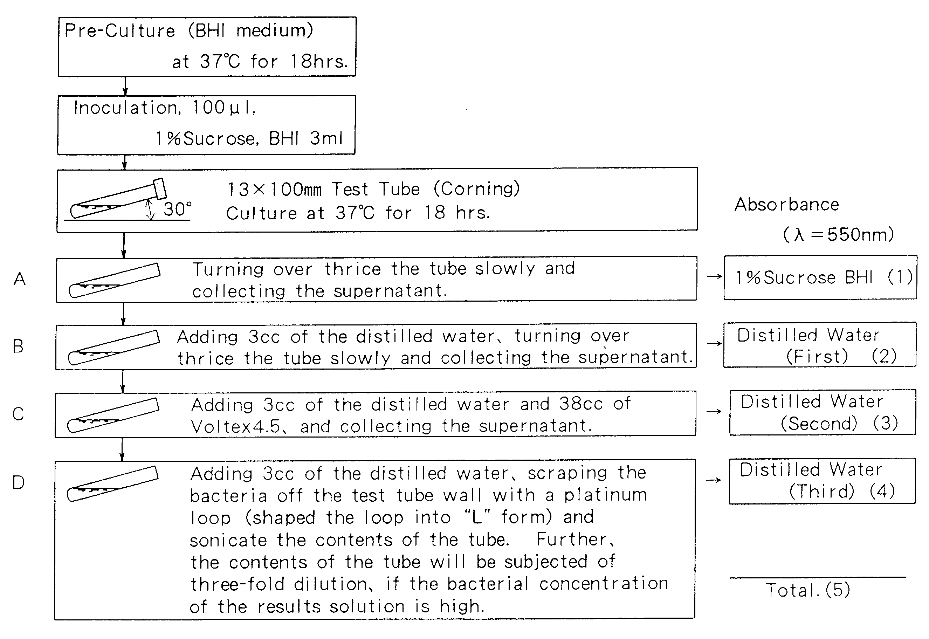

Since Streptococcus salivarius M-33 produced remarkable amounts of dextranase, as referred to the above, an experiment for eliminating the dental plaque by Streptococcus salivarius M-33 was conducted according to the method shown in the accompanying drawing.

First of all, Streptococcus sobrinus 6715 which is a cariogenic bacteria was pre-cultured on Brain Heart Infusion (Difco) medium at 37°C for 18 hours. A 50µl pre-culture was inoculated into 3ml of Brain Heart Infusion medium supplemented with 1% sucrose. As shown in the drawing, the culture was conducted at 37°C by a static culture wherein 13 x 100 mm test tube (Corning) is sloped 30 degrees. After 18 hours, the amount of the insoluble glucan (dental plaque) adhered to the test tube wall was read in accordance with the order of A, B, C and D of the drawing. The data in the following Table 2 are determined spectrophotometrically as the density for the amount of insoluble glucan at 550nm.

**TABLE 2**

| Culture System | (1) | (2) | (3) | (4) | Total (5) | (3)/(5) (loose) | (4)/(5) (firm) | $\frac{\text{[(}}{\text{(5}}$ ⁽^{***}⁾ |
|---|---|---|---|---|---|---|---|---|
| Mono-culture of 6715 | 0 | 0.0108 | 0.257 | 0.734 | 1.002 | 24.90 | 74.04 | 98.94 |
| co-culture of 6715 and M-33 | 0.26 | 0.27 | 0.91 | 0.50 | 1.93 | 47.36 | 25.72 | 73.08 |
| co-culture of 6715 and G8326 | 0.36 | 0.18 | 0.54 | 0.72 | 1.80 | 30.02 | 40.61 | 70.63 |

| | | | | | | | | |
|---|---|---|---|---|---|---|---|---|
| [***] Total amount of the Dental Plaque | | | | | | | | |

The insoluble glucan of columns 1 and 2 of Table 2 were obtained by "rinse" and are not considered as dental plaque. The insoluble glucan of the column 3 is the fragile dental plaque, obtained by "vortex" and referred to as "loose". The insoluble glucan of the column 4 is from dental plaque obtained by sonication (1 min) and referred to as "firm"

The results shown in Table 2 which indicate the amount of dental plaque formed in the experiment of the mono-culture of Streptococcus sobrinus 6715 (cariogenic bacteria) shows that the most of the dental plaque produced thereby is "firm".

The examination with regard to the co-culture of Streptococcus sobrinus 6715 and Streptococcus salivarius M-33 was conducted under the same conditions as the control, by culturing and treating a medium inoculated with 50µl of Streptococcus salivarius M-33 and 50µl of Streptococcus sobrinus 6715. As shown in Table 2, the dental plaque obtained in this case contained much dental plaque obtained by "rinse" and "loose" dental plaque and, on the other hand, contained less "firm" dental plaque. A similar tendency is observed in the co-culture of Streptococcus salivarius G8326 and Streptococcus sobrinus 6715 of cariogenic bacteria.

From the results aforementioned, it was found that in the co-culture of bacteria which causes dental caries and Streptococcus salivarius M-33 or Streptococcus salivarius G8326 either of which produce dextranase, "firm" dental plaque was reduced and "loose" plaque increased. In other words, in the case of the co-culture of a bacteria which causes the dental caries and Streptococcus salivarius G8326, although dental plaque is produced, the fragile proportion of and removable glucan, wherein the alpha-1,6 bond thereof constitutes dental plaque broken by the dextranase activity, would seem to be increased.

### Example 3: The Relation between the Degree of the Dextranase Productivity of Streptococcus salivarius and the Shape of the Colony

In view of the results of Examples 1 and 2, degradation effect of dental plague by the strains Streptococcus salivarius having high dextranase productivity was confirmed. Therefore an experiment to specify the sub-group of Streptococcus salivarius having the features aforementioned was conducted.

Each strain of Streptococcus salivarius as used in Example 1 was inoculated onto Mitis-Salivarius Agar Plate (Difco), which had been added 1% Chapman solution and the plates were then incubated at 37°C. After about 30 hours incubation, most of the strains formed the typical large smooth colony as disclosed in the right column of Table 1. After 48 hours incubation of continuous cultivation, the center of the colony of Streptococcus salivarius M-33 or Streptococcus salivarius G8326 had sunk and the colony gradually formed into a so called crater-form. In strains having weak dextranase productivity, such phenomenon was not observed. In contrast thereto, the strain having no dextranase productivity formed an untypical rough colony.

Accordingly, a correlation was established between high dextranase productivity and the shape of the colony, those strains which produce dextranase give a crater-form colony after 48 hours or more incubation.

The correlation between the degree of the dextranase productivity and the shape of the colony can be understood easily for the following reasons. Generally, Streptococcus salivarius produce, as an extracellular polysaccharide, the water-soluble fructan (levan) and the water-insoluble glucan (dextran). Since the polysaccharide was produced according to cell multiplication, the colony thereof would be formed into the raised, glossy smooth colony. Since the dextranase productivity of the most of Streptococcus salivarius strains are weak as shown in the left column of Table 1, the dextranase would not be degraded by the prolonged cultivation and the shape of the colony would be unchanged. In contrast thereto, although Streptococcus salivarius M-33 or Streptococcus salivarius G8326 produce smooth colony at the early stage, prolonged cultivation would form a crater shaped colony because the dextran was degraded in spite of cell multiplication stopping.

The present invention provides a composition, such as a food or medicine, containing lactic acid bacteria in which the composition has the novel property of "dental caries prevention" together with the conventional property of "regulating the intestinal condition".

## Claims

1. A composition comprising a live and native Streptococcus salivarius which is capable of staying in the oral cavity, of producing dextranase and of producing in a medium a crater-form colony.

2. A composition as claimed in Claim 1 in which the Streptococcus salivarius is Streptococcus salivarius M-33 (FERM BP-3885).

3. A live and native Streptococcus salivarius which is capable of staying in the oral cavity, of producing in a medium a crater-form colony and of producing dextranase for use in preventing dental caries.

4. A live and native Streptococcus salivarius M-33 (FERM BP-3885) for use in preventing dental caries.

5. The use of a live and native Streptococcus salivarius which is capable of staying in the oral cavity, of producing in a medium a crater-form colony and of producing dextranase for the manufacture of a composition for oral ingestion to remove dental plaque.

6. A live and native Streptococcus salivarius M-33 (FERM BP-3885) for the manufacture of a composition for oral ingestion to remove dental plaque.

7. A method for producing the composition comprising live and native Streptococcus salivarius which is capable of staying in the oral cavity, of producing in a medium a crater-form colony and of producing dextranase comprising:
(a) culturing, in a medium, the Streptococcus salivarius,
(b) selecting the strain which produces a crater-form colony, and
(c) multiplying the selected strain.

## Patentansprüche

1. Zusammensetzung, die einen lebenden und nativen Streptococcus salivarius umfaßt, der in der Lage ist, die Mundhöhle zu besiedeln, Dextranase zu produzieren und in einem Medium eine trichterförmige Kolonie zu bilden.

2. Zusammensetzung nach Anspruch 1, in der es sich bei dem Streptococcus salivarius um Streptococcus salivarius M-33 (FERM BP-3885) handelt.

3. Lebender und nativer Streptococcus salivarius, der in der Lage ist, die Mundhöhle zu besiedeln, in einem Medium eine trichterförmige Kolonie zu bilden und Dextranase zu produzieren, zur Verwendung für die Verhütung von Zahnkaries.

4. Lebender und nativer Streptococcus salivarius M-33 (FERM BP-3885), der zur Verhütung von Zahnkaries Verwendung findet.

5. Verwendung eines lebenden und nativen Streptococcus salivarius, der in der Lage ist, die Mundhöhle zu besiedeln, in einem Medium eine trichterförmige Kolonie zu bilden und Dextranase zu produzieren, zur Herstellung einer Zusammensetzung zur peroralen Aufnahme zwecks Entfernung von Zahnbelag.

6. Lebender und nativer Streptococcus salivarius M-33 (FERM BP-3885) für die Herstellung einer Zusammensetzung zur peroralen Aufnahme zwecks Entfernung von Zahnbelag.

7. Verfahren zur Herstellung der Zusammensetzung, die einen lebenden und nativen Streptococcus salivarius enthält, der in der Lage ist, die Mundhöhle zu besiedeln, in einem Medium eine trichterförmige Kolonie zu bilden und Dextranase zu produzieren, das folgende Schritte umfaßt:
(a) Kultivieren des Streptococcus salivarius in einem Medium,
(b) Auswählen des Stammes, der eine trichterförmige Kolonie bildet, und
(c) Vermehren des ausgewählten Stammes.

## Revendications

1. Une composition comprenant un Streptococcus salivarius, vivant et natif, capable de séjourner dans cavité orale, de produire de la dextranase et de produire dans un milieu une colonie en forme de cratère.

2. Une composition selon la revendication 1, dans laquelle le Streptococcus salivarius est le Streptococcus salivarius M-33 (FERM BP-3885).

3. Un Streptococcus salivarius vivant et natif capable de séjourner dans la cavité orale, de produire dans un milieu une colonie en forme de cratère et de produire de la dextranase, destinée à être utilisée dans la prévention des caries dentaires.

4. Un Streptococcus salivarius M-33 vivant et natif (FERM BP-3885), destiné à être utilisé dans la prévention des caries dentaires.

5. L'utilisation d'un Streptococcus salivarius vivant et natif capable de séjourner dans la cavité orale, de produire dans un milieu une colonie en forme de cratère et de produire de la dextranase pour la fabrication d'une composition destinée à l'ingestion orale afin d'éliminer la plaque dentaire.

6. Un Streptococcus salivarius M-33 vivant et natif (FERM BP-3885) pour la fabrication d'une composition destinée à l'ingestion orale pour éliminer la plaque dentaire.

7. Un procédé de production de la composition comprenant un Streptococcus salivarius vivant et natif capable de séjourner dans la cavité orale, de produire dans un milieu une colonie en forme de cratère et de produire de la dextranase, comprenant :
(a) la mise en culture dans un milieu du Streptococcus salivarius,
(b) la sélection de la souche produisant une colonie en forme de cratère, et
(c) la multiplication de la souche sélectionnée.
